# FASCICULE DE BREVET EUROPEEN

(11) **EP 0 428 429 B1**
(45) Date de publication et mention de la délivrance du brevet: **28.09.1994**
(21) Numéro de dépôt: 90403072.3
(22) Date de dépôt: 30.10.1990
(51) Int. Cl.: C07C 51/27

(54) **Procédé de fabrication industrielle en continu d'une solution aqueuse d'acide glyoxylique**
Verfahren zur kontinuierlichen Industrieherstellung der wässrigen Glyoxylsäurelösung
Process for a continuous industrial preparation of an aqueous solution of glyoxylic acid

(30) Priorité: 16.11.1989 FR 8915036
(43) Date de publication de la demande: 22.05.1991
(73) Titulaire: SOCIETE FRANCAISE HOECHST, 92800 Puteaux (FR)
(72) Inventeur: Schouteeten, Alain, F-95400 Ezanville (FR); Alarcon, Jean-Michel, F-60350 Trosly Breuil (FR)
(74) Mandataire: Rinuy, Santarelli

(56) Documents cités:
- DE-B- 1 002 309
- FR-A- 2 516 506
- PATENT ABSTRACTS OF JAPAN, vol. 9, no. 74 (C-273)(1797), 3 Avril 1985; & JP-A-59 210 028

## Description

La présente invention concerne un nouveau procédé de fabrication industrielle en continu d'une solution aqueuse d'acide glyoxylique.

On sait depuis longtemps préparer des solutions aqueuses d'acide glyoxylique par oxydation nitrique de solutions aqueuses de glyoxal (brevets français N° 1 326 605, 2 372 141, 2 516 506, brevets allemands N° 932 369, 933 987, 1 002 309, demandes de brevet japonais N° 73-103517, 76-29441, 76-80821, 77-105121, 80-129240 et brevet de la République Démocratique Allemande N° 128 233).

Tous ces procédés sont réalisés avec de l'acide nitrique, en présence éventuellement soit d'un acide minéral fort, tel que l'acide chlorhydrique (FR 2 516 506 et JP Kokai 73-103517), soit d'un sel soluble de cobalt (DD 128233), soit enfin d'oxygène moléculaire (JP Kokai 76-29441, 76-80821, 77-105121, 82-55698, 80-129240) et ils fournissent des solutions aqueuses d'acide glyoxylique contenant, outre du glyoxal et de l'acide nitrique résiduels dos quantités relativement importantes d'oxydes d'azote et d'acide oxalique qu'il est nécessaire d'éliminer par des traitements subséquents laborieux (cf. notamment aux brevets français N° 2 297 830 et 2 552 426).

La présence de glyoxal et d'acide nitrique résiduels est particulièrement gênante, aussi leur élimination doit elle être effectuée de manière à obtenir une concentration résiduelle en glyoxal inférieure à 2 % molaire par rapport à l'acide glyoxylique et une concentration en acide nitrique inférieure à 0,5 % molaire par rapport à l'acide glyoxylique.

Le brevet français N° 2 516 506 enseigne un procédé d'oxydation de solutions aqueuses de glyoxal en solution aqueuse d'acide glyoxylique par un agent oxydant obtenu à partir d'acide nitrique et d'un acide fort non oxydant présent à une concentration pondérale de 6 à 40 % dans le mélange réactionnel.

Les solutions aqueuses d'acide glyoxylique issues de ce procédé offrent l'intérêt de présenter de faibles concentrations résiduelles en acide nitrique. Elles ne répondent toutefois pas totalement aux exigences du marché en raison de leurs concentrations trop élevées en glyoxal.

Or la Demanderesse a découvert un nouveau procédé de fabrication industrielle d'une solution aqueuse d'acide glyoxylique obviant a ces inconvénients.

Le procédé selon la présente invention qui consiste à soumettre une solution aqueuse de glyoxal à une oxydation nitrique en présence d'acide chlorhydrique est caractérisé en ce qu'il est réalisé en continu dans un milieu réactionnel contenant une concentration pondérale en acide nitrique supérieure à 10 % et en acide chlorhydrique comprise entre 5 et 6 % à l'aide de 0,80 ± 0,02 mole d'acide nitrique et 0,70 ± 0,05 mole d'acide chlorhydrique par mole de glyoxal mis en oeuvre.

Le procédé de la présente invention permet de transformer quasi quantitativement le glyoxal en un mélange d'acides glyoxylique et oxalique. Les pertes de glyoxal sont très faibles, toujours inférieures à 1 % et le glyoxal non transformé est toujours inférieur à 2 %. Quant à l'acide nitrique, il est entièrement consommé, si bien que les solutions aqueuses d'acide glyoxylique obtenues contiennent toujours moins de 0,1 % en poids d'acide nitrique résiduel. La sélectivité de l'oxydation du glyoxal en acide glyoxylique est comprise entre 75 et 80 %.

Le procédé selon la présente invention est mis en oeuvre de préférence à une température comprise entre 30 et 80°C. On opère notamment a une pression comprise entre 0,5 et 10 bars et avantageusement à une pression comprise entre 1 et 5 bars.

Le procédé selon la présente invention peut être réalisé par exemple dans une série de réacteurs agités en cascade, ou mieux, dans un ensemble constitué par un ou plusieurs réacteurs agités suivi d'un ou plusieurs réacteurs pistons.

Avantageusement, on utilise soit trois réacteurs agités en cascade, soit de préférence un réacteur agité suivi de deux réacteurs pistons. L'alimentation s'effectue en continu dans le premier réacteur et avantageusement, on lave les gaz issus du procédé par la solution aqueuse de glyoxal d'alimentation. En sortie du dernier réacteur, le milieu réactionnel est refroidi à la température ambiante puis on élimine l'acide oxalique formé par cristallisation et les eaux mères contenant l'acide glyoxylique formé, l'acide chlorhydrique et des traces d'acide oxalique et d'acide nitrique sont soumises à deux électrodialyses successives qui permettent d'extraire du milieu réactionnel, d'abord l'acide chlorhydrique qui est recyclé, puis l'acide oxalique résiduel qui est recyclé dans le milieu réactionnel issu du dernier réacteur.

Le milieu réactionnel en sortie du deuxième électrodialyseur est soit utilisé tel quel, soit concentré sous pression réduite à la concentration désirée en acide glyoxylique.

Pour l'alimentation, on utilise de préférence des solutions aqueuses commerciales de glyoxal, d'acide chlorhydrique et d'acide nitrique éventuellement diluées avec de l'eau. L'oxydation selon le procédé de l'invention est rapide : habituellement elle nécessite moins de deux heures et elle est avantageusement conduite dans des réacteurs successifs chauffés à des températures identiques ou de plus en plus élevées.

Préférentiellement, l'oxydation est commencée à 45°C, puis elle est terminée à 55°C.

Les solutions aqueuses d'acide glyoxylique sont notamment couramment utilisées pour accéder par exemple à la vanilline à partir du gaïacol ou à l'acide parahydroxymandélique à partir du phénol.

L'exemple suivant illustre l'invention sans toutefois la limiter.

### Exemple

On alimente en continu un réacteur agité maintenu a 45 ± 1°C, suivi de deux réacteurs pistons en cascade, maintenus à 55°C, avec :
- 29 020 g/h d'une solution aqueuse de glyoxal à 20 % en poids, soit 100 moles/h de glyoxal,
- 13 322 g/h d'acide chlorhydrique à 20 % en poids, soit 73 moles/h de chlorure d'hydrogène,
- 7 441 g/h d'acide nitrique à 69 % en poids, soit 81,5 moles/h d'acide nitrique à 100 %.

Ce mélange réactionnel reste 12 minutes dans le réacteur agité, puis 35 minutes dans le premier réacteur piston et enfin 45 minutes dans le deuxième réacteur piston, puis le mélange réactionnel est refroidi. L'acide oxalique formé cristallise et après élimination des cristaux d'acide oxalique par filtration, le milieu réactionnel est soumis a deux électrodialyses successives pour éliminer l'acide chlorhydrique puis l'acide oxalique résiduel. L'acide chlorhydrique isolé dans le premier électrodialyseur est recyclé en tête du procédé et la solution aqueuse d'acide oxalique contenant des traces d'acide chlorhydrique et d'acide glyoxylique est recyclée en sortie du deuxième réacteur piston.

En sortie du deuxième réacteur piston, on obtient 47 391 g/h d'une solution aqueuse contenant pondéralement 12,11 % d'acide glyoxylique, soit 77,5 moles/h, 5,62 % d'acide chlorhydrique, soit 73 moles/h, 3,9 % d'acide oxalique, soit 20,5 moles/h, 0,18 % de glyoxal, soit 1,5 mole/h, 0,04 % d'acide nitrique, soit 0,3 mole/h et enfin 78,15 % d'eau. Parallèlement, il se dégage des gaz qui après lavage avec la solution aqueuse de glyoxal de départ ont un débit de 2 390 g/h et qui sont constitués par 92,6% d'oxyde d'azote, soit 73,8 moles/h, 1,2 % d'azote, soit 1 mole/h, 4,1 % de monoxyde de diazote, soit 2,2 moles/ h et 2,1 % de dioxyde de carbone, soit 1,1 mole/h.

Le rendement en acide glyoxylique s'établit à 77-78 % par rapport au glyoxal mis en oeuvre, le rendement en acide oxalique s'établit à 20-21 % par rapport au glyoxal et seulement 0,5 % du glyoxal est perdu à l'état de dioxyde de carbone.

Le taux de transformation du glyoxal est d'environ 98,5 %.

En sortie du deuxième électrodialyseur, la solution aqueuse d'acide glyoxylique contenant des traces infimes d'acide chlorhydrique, d'acide oxalique et d'acide nitrique est concentrée de manière à amener son titre à 40 % ou 50 % selon les demandes commerciales.

On peut remplacer l'acide nitrique à 69 % par de l'acide nitrique à 55 %. Pour ce faire, il suffit d'alimenter le réacteur avec une solution aqueuse soit de glyoxal, soit d'acide chlorhydrique, soit de ces deux produits légèrement plus concentrée. Ainsi, par exemple, si l'on alimente le réacteur avec 81,5 moles/h d'acide nitrique à 55 %, soit 9 335,5 g/h, on utilise 27 126 g/h d'une solution aqueuse de glyoxal à 21,4 %, soit 100 moles.

## Revendications

1. Procédé de fabrication d'une solution aqueuse d'acide glyoxylique par oxydation nitrique d'une solution aqueuse de glyoxal réalisée en présence d'acide chlorhydrique, caractérisé en ce que cette oxydation est effectuée en continu à l'aide de 0,80 ± 0,02 mole d'acide nitrique et 0,70 ± 0 05 mole d'acide chlorhydrique par mole de glyoxal dans un milieu réactionnel présentant une concentration pondérale comprise entre 5 et 6 % en acide chlorhydrique et supérieure à 10 % en acide nitrique.

2. Procédé selon la revendication 1, caractérisé en ce qu'il est mis en oeuvre à une température comprise entre 30 et 80°C.

3. Procédé selon la revendication 1 ou 2, caractérisé en ce qu'il est réalisé a une pression comprise entre 0,5 et 10 bars.

## Claims

1. Manufacturing process for an aqueous solution of glyoxylic acid by nitric oxidation of an aqueous solution of glyoxal carried out in the presence of hydrochloric acid, characterized in that this oxidation is effected continuously using 0.80 ± 0.02 mole of nitric acid and 0.70 ± 0.05 mole of hydrochloric acid per mole of glyoxal in a reaction medium which has a concentration by weight comprised between 5 and 6% of hydrochloric acid and higher than 10% of nitric acid.

2. Process according to claim 1, characterized in that it is used at a temperature comprised between 30 and 80°C.

3. Process according to claim 1 or 2, characterized in that it is carried out at a pressure comprised between 0.5 and 10 bars.

## Patentansprüche

1. Verfahren zur Herstellung einer wäßrigen Glyoxylsäurelösung durch Stickstoffoxidation einer wäßrigen Glyoxallösung in Gegenwart von Salzsäure, dadurch gekennzeichnet, daß diese Oxidation kontinuierlich mittels 0,80 ± 0,02 Mol Salpetersäure und 0,70 ± 0,05 Mol Salzsäure pro Mol Glyoxal in einem Reaktionsmedium erfolgt, das eine Gewichtskonzentration von 5 bis 6 % Salzsäure und mehr als 10 % Salpetersäure aufweist.

2. Verfahren nach Anspruch 1, dadurch gekennzeichnet, daß man es bei einer Temperatur zwischen 30 und 80°C durchführt.

3. Verfahren nach Anspruch 1 oder 2, dadurch gekennzeichnet, daß man es bei einem Druck zwischen 0,5 und 10 bar durchführt.
